# EUROPEAN PATENT SPECIFICATION

(11) **EP 1 171 634 B1**
(45) Date of publication and mention of the grant of the patent: **26.10.2005**
(21) Application number: 00920202.9
(22) Date of filing: 07.04.2000
(51) Int. Cl.: C12Q 1/68, C12P 19/34, C07H 21/04

(54) **AMPLIFICATION AND SEQUENCING PRIMER PAIRS AND USE THEREOF**
AMPLIFIKATION UND SEQUENZIERUNG VON PRIMER-PAAREN UND DEREN VERWENDUNG
PAIRES D'AMORCES D'AMPLIFICATION ET DE SEQUENCAGE ET LEUR EMPLOI

(30) Priority: 08.04.1999 US 128378 P
(43) Date of publication of application: 16.01.2002
(73) Proprietor: GEN-PROBE INCORPORATED, San Diego, CA 92121 (US)
(72) Inventor: BROWN, Bob, D., Encinitas, CA 92024 (US)
(74) Representative: Van Malderen, Joelle
(86) International application number: PCT/US2000/009230
(87) International publication number: WO 2000/061807

(56) References cited:
- WO-A-91/15601
- US-A- 5 104 792
- US-A- 5 451 503
- US-A- 5 612 199
- US-A- 5 627 032
- US-A- 5 650 271
- US-A- 5 683 879
- US-A- 5 981 179
- US-A- 6 027 863
- "CHEMICAL ABSTRACTS + INDEXES, AMERICAN CHEMICAL SOCIETY. COLUMBUS, US" CHEMICAL ABSTRACTS + INDEXES, AMERICAN CHEMICAL SOCIETY. COLUMBUS, US, XP000376987 ISSN: 0009-2258

## Description

### Background of the Invention

### Field of the Invention

The present invention relates to two-component combinatorial PCR primer pairs and their use in amplifying and sequencing nucleic acid sequences.

### Description of the Related Art

The polymerase chain reaction (PCR) is a DNA amplification method in which oligonucleotide primers complementary to opposite ends of a gene sequence are used to amplify the sequence by repeated cycles of denaturation, annealing and extension in the presence of a DNA polymerase having activity at elevated temperatures. However, this method relies on knowing the sequences at the opposite ends of the gene in order to design primers which can hybridize to these regions. Thus, each time a gene is to be amplified, different oligonucleotide primers must be synthesized. Alternatively, if one wishes to have primers on hand, a standing library of millions or billions of conventional oligonucleotides would be needed to successfully target and amplify each of the approximately 100,000 human genes. An ordered library of millions of PCR primers is beyond the chemical, physical and organizational tools currently available.

There is a need for a pre-made PCR primer library containing reasonable numbers of primers capable of binding to every target sequence present in the genome. The present invention addresses this need.

### Summary of the Invention

The present invention relates to a set of primers for amplification or sequencing of a target polynucleotide comprising an oligonucleotide primer pair and a universal primer as recited in claim 1.

One embodiment of the present invention is an amplification primer pair comprising an oligonucleotide anchor and primer, the anchor having a nucleic acid chemistry which is not a substrate for reverse transcriptases or DNA polymerases, and/or having a 3'-end which is not capable of priming DNA synthesis; the primer having a nucleic acid chemistry that is a substrate for reverse transcriptases or DNA polymerases; the anchor and the primer each including a region of complementary nucleotides which are capable of associating with each other to form a stem structure which includes a region which is complementary to a universal primer. Preferably, the anchor sequence and the stem regions are connected by a flexible linker. In one aspect of this preferred embodiment, the flexible linker is selected from the group consisting of polyethylene glycol, polypropylene glycol, polyethylene, polypropylene, polyamides and polyesters. Preferably, the primer comprises a tail region which extends beyond the length of the stem region of the anchor. In one aspect of this preferred embodiment, the primer comprises one or more modified bases. In another aspect of this preferred embodiment, the anchor comprises one or more modified backbone linkages. Advantageously, the anchor and said primer are each between 6 and 24 bases in length. Preferably, the primer pair is in association with a universal primer which is complementary to a region of the stem structure.

Another embodiment of the present invention is a sequencing primer pair comprising an oligonucleotide anchor and primer, the anchor having a nucleic acid chemistry which is not a substrate for reverse transcriptases or DNA polymerases, and/or having a 3'-end which is not capable of priming nucleic acid synthesis; the primer having a nucleic acid chemistry that is a substrate for reverse transcriptases or DNA polymereses; and the anchor and primer each including a region of complementary nucleotides which are capable of associating with each other to form a stem structure. Preferably, the anchor sequence and the stem regions are connected by a flexible linker. In one aspect of this preferred embodiment, the flexible linker is selected from the group consisting of polyethylene glycol, polypropylene glycol, polyethylene, polypropylene, polyamides and polyesters. Preferably, the primer comprises a tail region which extends beyond the length of the stem region of the anchor. In one aspect of this preferred embodiment, the primer comprises one or more modified bases. In another aspect of this preferred embodiment, the anchor comprises one or more modified backbone linkages. Advantageously, the anchor and said primer are each between 6 and 24 bases in length. Preferably, the primer pair is in association with a universal primer which is complementary to a region of the stem structure.
The present invention also provides a method for amplifying a nucleic acid sequence, comprising the steps of: combining a nucleic acid molecule with a forward anchor (FA), forward primer (FP), reverse anchor (RA), reverse primer (RP), forward universal primer (FUP) and reverse universal primer (RUP), wherein the FA/FP form a first primer pair and the RA/RP form a second primer pair via association of their complementary stem regions, wherein the FUP is complementary to the FA/FP stem region, and wherein the RUP is complementary to the RA/RP stem region wherein the primer pairs are selected to flank the RNA sequence; and amplifying said nucleic acid sequence via enzyme-mediated amplification. Advantageously, the nucleic acid sequence encodes a therapeutic gene product. Preferably, the nucleic acid sequence is DNA or RNA. In one aspect of this preferred embodiment, the enzyme-mediated amplification is PCR amplification

### Brief Description of the Drawings

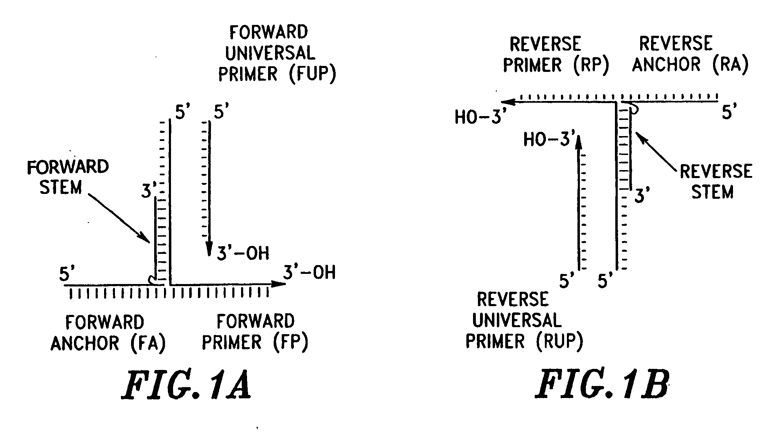

Figures 1A and 1B are schematic diagrams of forward and reverse amplification primer pairs, respectively. Each primer pair is generated from a library of individual oligonucleotides which are combined to form the primer pairs shown in the figure. One component of the primer pair is called the anchor (forward anchor = FA; reverse anchor = RA), and the other component is called the primer (forward primer = FP; reverse primer = RP). The two components are non-covalently bonded by a region of complementary oligonucleotides called the stem. The anchors may be linked to the stems by a linker. The forward universal primer (FUP) and reverse universal primer (RUP) are complementary to the forward and reverse stems, respectively. The primer pairs are tailored to amplify a specific gene sequence by choosing the appropriate individual oligonucleotide sequences. The reverse primer primes first strand cDNA synthesis, and the forward primer primes second strand cDNA synthesis.

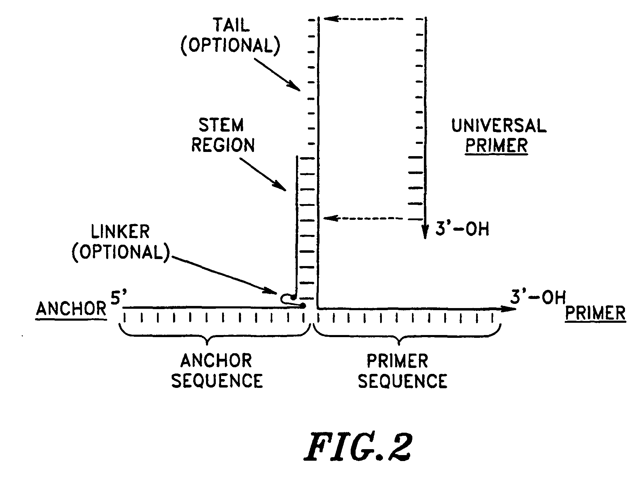

Figure 2 is a schematic diagram of an amplification primer pair showing the optional linker, stem, optional tail and universal primer.

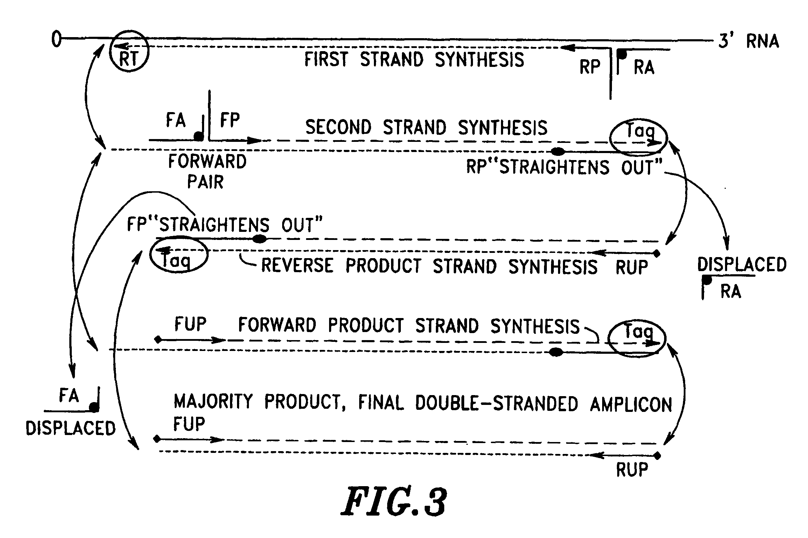

Figure 3 is a schematic diagram of the method of the invention using amplification primer pairs generated from a library of individual oligonucleotides. RA and FA are oligonucleotides comprising a plurality of modified bases and linkages for maximal binding affinity and are not capable of priming the nucleic acid amplification reaction (i.e. do not contain a 3'-OH group). These components may include a flexible linker. RP and FP serve as the amplification primers and are incorporated into the resulting DNA molecule. First strand cDNA synthesis is primed by RP, and second strand cDNA synthesis is primed by FP. FA and RA are displaced during the amplification reaction. RUP and FUP are universal primers complementary to a portion of RP and FP, respectively, which are incorporated into the amplified cDNA. RUP and FUP primer reverse and forward product strand synthesis, respectively. The final product is a double stranded amplicon containing RUP and FUP.

### Detailed Description of the Preferred Embodiments

The present invention provides amplification and sequencing primer pairs for use in amplifying or sequencing any DNA sequence of interest. These primer pairs can be prepared quickly, using a feasible number of pre-synthesized components present in a primer library, and are used for nucleic acid synthesis and detection. The library of components is suitable for forming any desired primer pair on demand. To amplify any desired nucleic acid sequence, two amplification primer pairs are used, each being generated from individual oligonucleotide components. All possible 6-mer, 8-mer, 12-mer or other length oligonucleotides are synthesized by conventional automated DNA sequencing and include a region which is complementary to a second set of 6-mers, 8-mers, 12-mers (or other length oligomers) so that when two such primers are combined *in vitro*, they self-assemble via hydrogen bonding of their complementary regions (Watson-Crick base pairing). In one embodiment of the invention, the anchor and primer oligonucleotides are each between 6 and about 24 bases in length. All possible 8-mers, for example, can be represented in a modestly-sized library of 4⁸ (65,536) oligonucleotides. All possible 16-mers, on the other hand, would require an enormous library of 4¹⁶ (4 x 10⁸) oligonucleotides.

The term "oligonucleotide" refers to a molecule consisting of DNA, RNA or DNA/RNA hybrids.

The term "oligonucleotide analog" refers to a molecule comprising an oligonucleotide-like structure, for example having a backbone and a series of bases, wherein the backbone and/or one or more of the bases can be other than the structures found in naturally-occurring DNA and RNA. "Non-natural" oligonucleotide analogs include at least one base or backbone structure that is not found in natural DNA or RNA. Exemplary oligonucleotide analogs include, but are not limited to, DNA, RNA, phosphorothioate oligonucleotides, peptide nucleic acids (PNA), methoxyethyl phosphorothioates, oligonucleotide containing deoxyinosine or deoxy 5-nitroindole, and the like.

The term "backbone" as used herein refers to a generally linear molecule capable of supporting a plurality of bases attached at defined intervals. Preferably, the backbone will support the bases in a geometry conducive to hybridization between the supported bases of a target polynucleotide.

The term "non-naturally occurring base" refers to a base other than A, C, G, T and U, and includes degenerate and universal bases as well as moieties capable of binding specifically to a natural base or to a non-naturally occurring base. Non-naturally occurring bases include, but are not limited to, propynylcytosine, propynyluridine, diaminopurine, 5-methylcytosine, 7-deazaadenosine and 7-deazaguanine.

The term "universal base" refers to a moiety that may be substituted for any base. The universal base need not contribute to hybridization, but should not significantly detract from hybridization. Exemplary universal bases include, but are not limited to, inosine, 5-nitroindole and 4-nitrobenzimidazole.

The term "degenerate base" refers to a moiety that is capable of base-pairing with either any purine, or any pyrimidine, but not both purines and pyrimidines. Exemplary degenerate bases include, but are not limited to, 6H, 8H. 3,4-dihydropyrimido[4,5-c][1,2]oxazin-7-one ("P", a pyrimidine mimic) and 2-amino-6-methoxyaminopurine ("K", a purine mimic).

The term "target polynucleotide" refers to DNA or RNA, for example as found in a living cell, with which a primer pair is intended to bind or react.

The term "library" refers to a collection of components that can be joined to form a variety of different molecules. In the practice of the invention, a library comprises at least two sets of oligonucleotides, designed such that oligomers of the first set can couple to oligomers of the second set. This coupling preferably occurs spontaneously on addition of the two oligomers.

The term "flexible linker" as used herein refers to a reactive chemical group that is capable of covalently attaching a binding domain to a coupling moiety. These linkers relieve stress that might otherwise result from interposing the coupling moieties between two binding domains that bind to adjacent regions of target nucleic acid. The flexible linker is preferably selected to be flexible, hydrophilic, and of sufficient length that the bulk of the coupling moieties does not interfere with hybridization., RNase recognition, and/or RNase activity on the complex. The linker may be connected to the terminal base of the binding domain, or can be connected one or more bases from the end. Suitable flexible linkers are typically linear molecules in a chain of at least one or two atoms, more typically an organic polymer chain of 1 to 12 carbon atoms (and/or other backbone atoms) in length. Exemplary flexible linkers include polyethylene glycol, polypropylene glycol, polyethylene, polypropylene, polyamides, polyesters and the like.

The term "modified backbone linkage" refers to internucleoside linkages other than phosphodiester linkages. Examples of these linkages include phosphorothioates, phosphorodithioates, methylphosphonates, morpholinos, MMI, peptide nucleic acids (PNA), 3'-amidates and the like.

The term "stem" as used herein refers to the structure formed by coupling two oligonucleotide or oligonucleotide analog coupling moieties.

Referring to Figures 1A-1B, two primer pairs are used: a forward pair and a reverse pair. Each pair comprises an anchor and a primer which are bonded via complementary base pairing in the stem region. The sequences of FA, FP, RA and RP are chosen from a primer library on the basis of the exact nucleic acid sequence or gene sequence to be amplified. The FUP and RUP primer sequences are not gene-specific. These sequences of these primers are based on the common forward stem and reverse stem sequences, respectively. FUP hybridize to all or part of the FP stem region, and RUP hybridizes to all or part of the RP stem region. The "tail" region shown in Figure 2 is optional. The FA and FP associate via base hybridization of the forward stem sequence, and RA and RP associate in the same manner. The stem sequences are too long for the small, overlapping complimentary regions in the FUP and RUP to interfere. Any FA can form a stem (hybridize to form a short duplex region) with any FP due to complementary stem sequences. Stem formation is not possible between any forward and reverse library members. Thus, FA will only pair with FP, and RA will only pair with RP.

The anchor components of the primer pairs preferably contain one or more modified bases and/or degenerate bases and/or universal bases, and may also contain one or more modified backbone linkages, to result in maximum affinity binding to a nucleotide sequence of interest. The anchors can have any nucleic acid chemistry (modified bases and/or linkages) which are not substrates for reverse transcriptases or DNA polymerases, and/or do not contain a chemical group such as a 3'-OH group, which is capable of priming DNA synthesis. In contrast, the primer components are much more "DNA-like" and contain substantially naturally-occurring oligonucleotides (A, G, T, C, U) and phosphodiester linkages because they must serve as a template for reverse transcriptase and DNA polymerase. The primer components may comprise any nucleic acid chemistry (combination of natural bases, unnatural bases, phosphodiester linkages, modified backbone linkages) which acts as a substrate for reverse transcriptase and DNA polymerase. The ability of any desired oligonucleotide or oligonucleotide analog to act as a substrate for reverse transcriptase and DNA polymerase can be readily determined by a person of ordinary skill in the art.

The anchor sequence and primer sequence may be joined to the stem region by an optional flexible linker (Fig. 2). A linker is likely to be more appropriate for anchors to relieve strain, increase binding affinity and improve the priming/polymerase substrate activity of the assembled anchor/primer complexes. Primers can also comprise linkers, although one should be used that can maintain polymerase activity.

With knowledge of the sequence to be amplified, the appropriate complementary FA, FP, RA, and RP oligonucleotides are selected from the library and combined to form primers. The gene sequence-specific amplification primer pairs are mixed in complete amplification (e.g. PCR) reaction buffer, including the universal primers. The ratio of FAIFP and RA/RP is typically about 1 to 10 or less. Thus, FA/FP and RAIRP typically only catalyze first and second strand cDNA synthesis. RA/RP is used for first strand synthesis, while FA/FP is used for second strand synthesis (Fig. 3). In contrast, FUP and RUP sustain the amplification reaction and produce the majority of the final amplicon product. The FA and FP immediately combine only with each other via base hybridization of the forward stem sequence, and the RA and RP combine only with each other in the same manner.

The template RNA mixture to be amplified, typically total cellular RNA or poly(A)+ RNA, is added to the reaction and the target RNA is bound by the RAIRP complex based on the hybridization of the gene sequence-specific sequence formed by the RA/RP complex assembly. Typically, a combined reverse transcriptase/thermostable DNA polymerase enzyme mix (e.g. *Taq* polymerase) is added to the reaction which is incubated at a temperature suitable for reverse transcription of the RNA (generally 37.55°C) to produce the first cDNA strand. First-strand cDNA synthesis is primed by the RA/RP structure, and the 3'-OH group of RP is extended by reverse transcriptase. RP is consumed by the reaction and becomes the 5'-end of the first strand cDNA product (Fig. 3). RA is displaced by second-strand cDNA synthesis and does not participate in subsequence rounds of amplification.

Second strand cDNA synthesis is primed by FA/FP, and the 3'-OH of FP is extended by DNA polymerase (thermostabile or otherwise). FP is consumed by the reaction, and becomes the 5'-end of the second strand product (also known as the "coding" or "sense" strand). FA is displaced during final "non-sense" strand synthesis and does not participate in subsequent rounds of amplification.

A standard thermocycle program is used to amplify the double stranded DNA product. Briefly, the reaction temperature is raised to 90.95°C to denature the nucleic acids. The temperature is then lowered to 50-65°C to allow primers to hybridize to complementary priming sites, then the temperature is raised to about 68-74°C to allow the thermostabile DNA polymerase to extend hybridized primers along the template DNA molecules. The time for each temperature is between about 15 seconds and 2 minutes. These steps are then repeated between about 25 and 35 times to amplify double stranded DNA.

After the first denaturation cycle, the FA/FP complex hybridizes to the first strand cDNA and is extended by DNA polymerase to produce the second strand of the cDNA and yield a double stranded cDNA product. It is important to note that FUP and RUP do not match the initial RNA sequence, and thus will not participate in the reaction until after first and second strand cDNA synthesis has occurred.

First and second strand priming and synthesis, and final amplicon priming and synthesis, consume the FP and RP. Extension of the priming groups, typically 3'-OH, by the polymerase cause FP and RP to become covalently incorporated into the product cDNA ends. Incorporation of the FP and RP stem sequences as the 5' ends of the cDNA provides the priming site for FUP and RUP, respectively, to bind and prime subsequent cDNA amplification. FUP and RUP are consumed by the reaction to produce the final double stranded DNA amplicon (Fig. 3). In addition, FP, RP, FUP, and/or RUP may have attached "detection" moieties to facilitate detection and/or capture of the cDNA or final DNA amplification product(s). Suitable "detection" moieties include enzymatic labels, fluorescent labels and radiolabels.

A preformed library of oligonucleotide primers is provided which comprises a first set of oligonucleotide primers and a second set of oligonucleotide primers, the primers having stem regions that allow coupling of the primers to form an amplification primer pair.

By conceptually separating the amplification primer into two pieces, a comprehensive amplification primer library can be prepared in advance, rather than synthesizing a plurality of amplification primers as needed. This primer pair system is advantageous to a single primer system because of increased binding affinity. A complete library of every possible 17-mer oligonucleotide, using the four natural bases, would consist of 4" (or about 1.7 x 10¹⁰) molecules. By providing the primers in two components, for example a library of 8-mers and a library of 9-mers, assembled quickly as needed, the size of the library needed is reduced to 4⁸ + 4⁹, or 327,650 molecules. The required complexity of the library is still further reduced by substituting one or more universal or degenerate bases for some of the natural bases. Thus, for example, if the 9-mer library consists of 5 universal bases followed by 4 natural bases, the number of components drops to 4⁴ (256), and the total library size is reduced to 4⁸ + 4⁴, about 66,000 molecules. The library complexity can also be reduced by dividing the antisense molecule into three or more segments. It is possible to synthesize and maintain libraries of this size, and rapidly assemble any desired primer pairs without the need for custom, de novo synthesis of long oligomers. Thus, one embodiment of the invention is a library comprising at least two sets of oligomers, wherein oligomers are selected from each set and coupled as needed.

At least one of the binding domains comprises about 3 to about 24 bases, preferably about 6 to about 8 bases. For example, a library can be constructed having a set of 6-mer binding domains, each of which binds only a single 6-mer sequence, and a set of 8-mer binding domains, in which only four of the bases are sequence-specific, and the remaining bases are degenerate or universal. The first set contains a possible 4⁶ (4096) sequences, while the second set contains only 4⁴ (256) sequences (assuming 4 "specific" bases and 4 universal bases). By combining oligomers selected from the first and second sets as needed, 4¹⁰ (10⁶) different sequences can be generated using only 4,352 molecules. In contrast, a complete library of 14-mers would require 4¹⁴ (2.7 x 10⁸) molecules.

The first oligonucleotide of the two-component primer (primer component P) comprises only naturally-occurring phosphodiester linkages and may contain one or more modified bases (for example, universal and degenerated bases as defined below). The second oligonucleotide (anchor component A) is modified to ensure high affinity binding to the RNA target sequence and contains one or more modified bases and modified backbone linkages, for example phosphorothioates, deoxy phosphorothioates, 2'-O-substituted phosphodiesters and deoxy analogs, 2'-0-substituted phosphodiesters and deoxy analogs, 2'-0-substituted phosphorothioates and deoxy analogs, morpholino, peptide nucleic acids (PNA; see U. S. Patent No. 5,539,082), 2'-0-alkyl methylphosphonates, 3'-amidates, MMI, alkyl ethers (see Cook et al., U. S. 5,223,618) and others as described in Cook et al., U. S. 5,378,825, Sanghvi et al. (U. S. 5,489,677), Cook et al. (U.S. 5,541,307) and the like.

Universal bases suitable for use in the present invention include, but are not limited to, deoxy 5-nitroindole, deoxy 3-nitropyrrole, deoxy 4-nitrobenzimidazole, deoxy nebularine, deoxyinosine, 2'-OMe inosine, 2'-OMe 5-nitroindole, 2'-OMe 3-nitropyrrole, 2'-F inosine, 2'-F nebularine, 2'-F 5-nitroindole, 2'-F 4-nitrobenzimidazole, 2'-F 3-nitropyrrole, PNA-5-introindole, PNA-nebularine, PNA-inosine, PNA-4-nitrobenzimidazole, PNA-3-nitropyrrole, morpholino-5-nitroindole, morpholino-nebularine, morpholino-inosine, morpholino-4-nitrobenzimidazole, morpholino-3-nitropyrrole, phosphoramidate-5-nitroindole, phosphoramidate-nebularine, phosphoramidate-inosine, phosphoramidate-4-nitrobenzimidazole, phosphoramidate-3-nitropyrrole, 2'-0-methoxyethyl inosine, 2'-O-methoxyethyl nebularine, 2'-0-methoxyethyl 5-nitroindole, 2'-0-methoxyethyl 4-nitro-benzimidazole, 2'-0-methoxyethyl 3-nitropyrrole, deoxy Rₚ MP-5-nitroindole dimer and 2'-OMe R, MP-5-nitroindole dimer.

Degenerate bases suitable for use in the present invention include, but are not limited to, deoxy P (A&G), deoxy K (U&C), 2'-OMe 2-aminopurine (U&C), 2'-OMe P (G&A), 2'-OMe K (U&C), 2'-F-2-aminopurine (U&C), 2'-F P (G&A), 2'-F K (U&C), PNA-2-aminopurine (U&C), PNA-P (G&A), PNA-K (U&C), morpholino-2-aminopurine (U&C), morpholino-P (G&A), morpholino-K (U&C), phosphoramidate-2-aminopurine (C&U), phosphoramidate-P (G&A), phosphoramidate-K (U&C), 2'-0-methoxyethyl 2-aminopurine (U&C), 2'-O-methoxyethyl P (G&A), 2'-O-methoxyethyl K (U&C), deoxy Rₚ MP-KP dimer, deoxy Rₚ MP-PK dimer, deoxy Rₚ MP-Kk dimer, deoxy Rₚ MP-PP dimer, 2'-OMe Rₚ MP-KP dimer, 2'-OMe R, MP-PK dimer, 2'-OMe Rₚ MP-KK dimer and 2'-OMe Rₚ MP-PP dimer.

The coupling moieties are selected to join two oligomers from different sets by either covalent or non-covalent interaction, for example a non-covalent binding pair. The coupling moieties are preferably selected such that the coupling moiety present on oligonucleotides of the first set in a library do not couple with each other, but bind readily with coupling moieties on oligonucleotides of the second set, thus ensuring that the oligonucleotides couple in the intended orientation. In one embodiment, the coupling moieties are complementary oligonucleotides or oligonucleotide analogs.

The primer pairs of the invention can also be used for nucleic acid sequencing and are the same as those used for nucleic acid amplification, except that they do not include the reverse or forward universal primers shown in Figs. 1A and 1B. For example, an FA/FP anchor/primer pair as shown in Fig. 1A or an RA/RP anchor/primer pair as shown in Fig. 1B may be used to prime sequencing reactions which are then performed as is well known in the art. In addition, the stem structure of these sequencing primer pairs may or may not include a region which is complementary to a universal primer.

### Example 1

### Amplification of PKCα

In the sequences shown below, L - linker from Glen Research (spacer 18, cat. # 10-1918-90 or spacer 9, cat. # 10-1909-90), underlined lower case nucleotides are gene-specific sequences and underlined upper case nucleotides are stem sequence regions. In this example, all primers contain only unmodified DNA backbones, and contain some high-affinity modified bases, such as C-5 Propynyl-C and C-5-propynyl-U. Anchors contain 2'-OMe-modified sugar backbones and C-5-Propynyl-C. Gene-specific regions for this example range in total length from 12 bases (6 each from anchors and primers) to 24 bases (12 each from anchors and primers). The step region is 24 bases long, and the universal primers (FUP and RUP) are 19 or 14 bases long.

Total cellular or poly(A+) RNA (0.1-2,000 ng) is added in the presence of (per reaction):
10 mM Tris-HCl, pH 8.3
50 mM KCI
200 µM each dATP, dGTP, dCTP, dTTP
0.2-1.0 µM FUP
0.2-1.0 µM RUP
0.02-0.1 µM each gene-specific FA, FP, RA and RP
200 U/100 µl reverse transcriptase
200 U/100µl thermostabile DNA polymerase

PCR is performed using the parameters described above. The size of the final product is verified by 1% agarose gel electrophoresis. The amplified PCR fragment is cleaned and purified using a commercial PCR cleaning kit (e.g., Qiagen), and can be used for *in vitro* or *in vivo* transfection of cells or tissues.

Although the examples described herein refer to amplification primer pairs for PCR amplification of a nucleic acid sequence, these primer pairs can be used for any DNA or RNA polymerase priming activity. For example, these primers pairs can be used in non-thermocycling primer-dependent amplification technologies that are used to compete commercially with PCR, such as strand displacement amplification (SDA) (Walker, *PCR Methods Appl*. **3**:1-6, 1993; Spargo et al., *Mol. Cell. Probes* **10**:247-256, 1996), self-sustained sequence replication (3SR) and in situ seff-sustained sequence replication (IS-3SR) (Mueller et al., *Histochem. Cell Biol*. **108**:431-437, 1997), rolling circle amplification (RCA) (Lizardi et al., *Nature Genet*. **19**:225-232, 19981, and nucleic acid sequence based amplification (NASBA) (Heim et al., *Nucl. Acids Res*. **26**:2250-2251, 1998; Romano et al., *Immunol. Invest*. **26**:15-28, 1997).

While particular embodiments of the invention have been described in detail, it will be apparent to those skilled in the art that these embodiments are exemplary rather than limiting, and the true scope of the invention is that defined in the following claims.

## Claims

1. A set of primers for amplification or sequencing of a target polynucleotide comprising:
- an oligonucleotide-primer pair comprising an oligonucleotide anchor and primer, wherein said anchor has a nucleic acid chemistry which is not a substrate for a reverse transcriptase or a DNA polymerase, and/or has a 3'-end which is not capable of priming nucleic acid synthesis,
wherein said primer comprises a primer sequence region that hybridizes to a target nucleic acid sequence and has a nucleic acid chemistry that is a substrate for a reverse transcriptase or a DNA polymerase, and
wherein said anchor and said primer each include a region of complementary nucleotides that self-assemble with each other to form a stem structure when said anchor and said primer oligonucleotides are combined, said stem structure including a region which is complementary to a universal primer; and
- a universal primer which is complementary to said region of said stem structure that is complementary to a universal primer.

2. The set of primers of claim 1, wherein said stem region of said anchor sequence is connected to the remaining anchor sequence by a flexible linker.

3. The set of primers of Claim 2, wherein said flexible linker is selected from the group consisting of polyethylene glycol, polypropylene glycol, polyethylene, polypropylene, polyamides and polyesters.

4. The set of primers of any one of Claims 1-3, wherein said primer comprises a tail region which extends beyond the length of said stem region of said anchor.

5. The set of primers of any one of claims 1-4 wherein said primer pair comprises one or more oligonucleotide analog molecules.

6. The set of primers of any one of Claims 1-5, wherein said primer sequence region comprises one or more modified bases.

7. The set of primers of Claim 6, wherein said one or more modified bases is a universal base.

8. The set of primers of Claim 7, wherein said universal base is selected from the group consisting of inosine, 5-nitroindole, 3-nitropyrrole, and 4-nitrobenzimidazole.

9. The set of primers of Claim 6, wherein said one or more modified bases is a degenerate base.

10. The set of primers of Claim 9, wherein said degenerate base is selected from the group consisting of 6H, 8H-3, 4-dihydropyrimido[4,5-c] [1,2]oxazin-7-one ("P", a pyrimidine mimic) and 2-amino-6-methoxyaminopurine ("K", a purine mimic).

11. The set of primers of any one of Claims 1-10, wherein said anchor comprises one or more modified backbone linkages.

12. The set of primers of any one of Claims 1-11, wherein the sequences of said anchor and said primer are each between 6 and 24 nucleotide bases in length.

13. Use of a set of primers according to any one of Claims 1-12 for amplification or sequencing of a target polynucleotide sequence.

14. A method for amplifying or sequencing a target nucleic acid sequence, comprising the steps of:
- combining a forward anchor (FA), forward primer (FP), reverse anchor (RA), reverse primer (RP), forward universal primer (FUP) and reverse universal primer (RUP), wherein said FA and said FP self-assemble to form a first oligonucleotide-primer pair (FA/FP) and said RA and said RP self-assemble to form a second oligonucleotide-primer pair (RA/RP) via association of their complementary stem regions, wherein said FUP is complementary to the FA/FP stem region and said RUF is complementary to the RA/RP stem region, and wherein said oligonucleotide-primer pairs are selected on the basis of complementarity to said target nucleic acid sequence;
- combining said first and second oligonucleotide-primer pairs and said FUP and RUP with said target nucleic acid; and
- amplifying said target nucleic acid sequence via enzyme-mediated amplification.

15. The method of Claim 14, wherein said target nucleic acid sequence is DNA.

16. The method of claim 14 wherein said target nucleic acid sequence is RNA.

17. The method of Claim 16 wherein said amplification utilizes reverse transcriptase.

18. The method of any one of Claims 14-17, wherein said enzyme-mediated amplification is selected from the group consisting of PCR, SDA, 3SR, IS-3SR, NASBA, and polling circle amplification.

19. The method of claim 18 wherein said enzyme-mediated amplification is PCR amplification.

20. The method of claim 18 wherein said enzyme-mediated amplification is selected from the group consisting of 3SR, IS-3SR, and NASBA amplification.

21. The method of claim 18 wherein said enzyme-mediated amplification is selected from the group consisting of SDA and rolling circle amplification.

## Patentansprüche

1. Satz von Primern für die Verstärkung oder die Sequenzierung eines Zielpolynukleotids, welcher enthält:
- ein Paar eines Oligonukleotidprimers mit einem Oligonukleotidanker und Oligonukleotidprimer, in welchem der besagte Anker eine Nukleinsäurechemie aufweist, welche kein Substrat für eine reverse Transkriptase oder eine DNA Polymerase darstellt und / oder eine 3'-Endgruppe besitz, welche nicht in der Lage ist, eine Nukleinsäuresynthese zu starten, in welchem der besagte Primer einen Primersequenzbereich aufweist, welcher zu einer Zielnukleinsäuresequenz hybridisiert und eine Nukleinsäurechemie aufweist, welche ein Substrat für eine reverse Transkriptase oder eine DNA Polymerase darstellt und in welchem der besagte Anker und der besagte Primer jeweils einen Bereich von komplementären Nukleotiden aufweisen, welche sich von selbst miteinander zusammenbauen, um eine Stammstruktur zu bilden wenn die besagten Ankeroligonukleotide und die besagten Primeroligonukleotide kombiniert werden, wobei die besagte Stammstruktur einen Bereich umfasst, welcher komplementär zu einem Universalprimer ist; und
- einen Universalprimer, der komplementär zu dem besagten Bereich der besagten Stammstruktur ist, die komplementär zu einem Universalprimer ist.

2. Satz von Primern gemäß Anspruch 1, bei welchem der besagte Stammbereich der besagten Ankersequenz durch einer flexiblen Linker mit der übrig bleibenden Ankersequenz verbunden ist.

3. Satz von Primern gemäß Anspruch 2, bei welchem der besagte flexible Linker ausgewählt wird aus der Gruppe bestehend aus Polyethylenglykol, Polypropylenglykol, Polyethylen, Polypropylen, Polyamiden und Polyestern.

4. Satz von Primern gemäß irgendeinem der Ansprüche 1-3, bei welchem der besagte Primer einen Schwanzbereich aufweist, der sich über die Länge des besagten Stammbereiches des besagten Ankers hinaus erstreckt.

5. Satz von Primern gemäß irgendeinem der Ansprüche 1-4, bei welchem das besagte Paar der Primer eine oder mehrere Oligonukleotidanalog-Moleküle aufweist.

6. Satz von Primern gemäß irgendeinem der Ansprüche 1-5, bei welchem der besagte Bereich der Primersequenz eine oder mehrere modifizierte Basen aufweist.

7. Satz von Primern gemäß Anspruch 6, bei welchem die besagte eine oder die besagten mehreren der modifizierten Basen aus einer Universalbase bestehen.

8. Satz von Primern gemäß Anspruch 7, bei welchem die besagte Universalbase ausgewählt wird aus der Gruppe bestehend aus Inosin, 5-Nitroindol, 3-Nitropyrrol und 4-Nitrobenzimidazol.

9. Satz von Primern gemäß Anspruch 6, bei welchem die besagte eine oder die besagten mehreren der modifizierten Basen aus einer degenerierten Base bestehen.

10. Satz von Primern gemäß Anspruch 9, bei welchem die besagte degenerierte Base ausgewählt wird aus der Gruppe bestehend aus 6H, 8H-3, 4-Dihydropyrimido[4,5-c][1,2]oxazin-7-on ("P", ein mimisches Pyrimidin) und 2-Amino-6-methoxyaminopurin ("K", ein mimisches Purin).

11. Satz von Primern gemäß irgendeinem der Ansprüche 1-10, bei welchem der besagte Anker eine oder mehrere modifizierte Hauptkettenverbindungen aufweist.

12. Satz von Primern gemäß irgendeinem der Ansprüche 1-11, bei welchem die Sequenzen des besagten Ankers und des besagten Primers eine jede eine Länge von zwischen 6 und 24 Nukleotidbasen aufweist.

13. Verwendung eines Satzes von Primern gemäß irgendeinem der Ansprüche 1-12, für die Verstärkung oder die Sequenzierung einer Zielpolynukleotidsequenz.

14. Verfahren für die Verstärkung oder die Sequenzierung eines Zielnukleinsäuresequenz, welches als Stufen aufweist;
- ein Kombinieren eines Vorwärtsankers (FA), eines Vorwärtsprimers (FP), eines reversen Ankers (RA), eines reversen Primers (RP), eines Vorwärtsuniversalprimers (FUP) und eines reversen Universalprimers (RUP), wobei der besagte FA und der besagte FP sich von selbst zusammenbauen, um ein erstes Paar eines Oligonukleotidprimers (FA/FP) zu bilden und wobei der besagte RA und der besagte RP sich von selbst zusammenbauen, um ein zweites Paar eines Oligonukleotidprimers (RA/RP) zu bilden über eine Assoziierung ihrer komplementären Stammbereiche, wobei der besagte FUP komplementär zu dem FA/FP Stammbereich ist und der besagte RUP komplementär zu dem RA/RP Stammbereich ist, und wobei die besagten Paare des Oligonukleotidprimers ausgewählt werden auf der Basis der Komplementarität zu der besagten Zielnukleinsäuresequenz:
- ein Kombinieren der besagten ersten und zweiten der Paare eines Oligonukleotidprimers und der besagten (FUP) und (RUP) mit der besagten Zielnukleinsäure; und
- ein Verstärken der besagten Zielnukleinsäuresequenz über eine von einem Enzym vermittelte Verstärkung.

15. Verfahren gemäß Anspruch 14, bei welchem die besagte Zielnukleinsäuresequenz DNA ist.

16. Verfahren gemäß Anspruch 14, bei welchem die besagte Zielnukleinsäuresequenz RNA ist.

17. Verfahren gemäß Anspruch 16, bei welchem die besagte Verstärkung die reverse Transkriptase benutzt.

18. Verfahren gemäß irgendeinem der Ansprüche 14-17, bei welchem die besagte von einem Enzym vermittelte Verstärkung ausgewählt wird aus der Gruppe bestehend aus einer PCR-, SDA-, 3SR-, IS-3SR-, NASBA- und Rollkreis-Verstärkung

19. Verfahren gemäß Anspruch 18, bei welchem die besagte von einem Enzym vermittelte Verstärkung eine PCR-Verstärkung ist.

20. Verfahren gemäß Anspruch 18, bei welchem die besagte von einem Enzym vermittelte Verstärkung ausgewählt wird aus der Gruppe bestehend aus einer 3SR-, IS-3SR- und NASBA-Verstärkung.

21. Verfahren gemäß Anspruch 18, bei welchem die besagte von einem Enzym vermittelte Verstärkung ausgewählt wird aus der Gruppe bestehend aus einer SDA- und einer Rollkreis-Verstärkung.

## Revendications

1. Jeu d'amorces pour l'amplification ou le séquençage d'un polynucléotide cible comprenant:
- une paire d'amorces comprenant un oligonucléotide d'ancrage et une amorce, où ledit ancrage possède une structure proprement dite ne pouvant être un substrat pour une transcriptase inverse ou une ADN polymérase, et/ou possède une extrémité 3' qui n'est pas capable d'amorcer une synthèse d'acide nucléique,
où ladite amorce proprement dite comprend une région qui s'hybride à une séquence d'acide nucléique cible et possède une structure qui est un substrat pour une transcriptase inverse ou une ADN polymérase, et
où ledit ancrage et ladite amorce proprement dite incluent chacun une région de nucléotides complémentaires qui s'auto-assemblent pour former une structure-tige lorsque les oligonucléotides dudit ancrage et de ladite amorce sont combinés, ladite structure-tige incluant une région qui est complémentaire à une amorce universelle; et
- une amorce universelle qui est complémentaire à ladite région de ladite structure-tige.

2. Jeu d'amorces selon la revendication 1, dans lequel ladite région impliquée dans la structure-tige de ladite séquence d'ancrage est reliée à la séquence d'ancrage restante par un lieur flexible.

3. Jeu d'amorces selon la revendication 2, dans lequel ledit lieur flexible est choisi dans le groupe constitué de polyéthylène glycol, de polypropylène glycol, de polyéthylène, de polypropylène, de polyamides et de polyesters.

4. Jeu d'amorces selon l'une quelconque des revendications 1-3, dans lequel ladite amorce comprend une queue qui s'étend au-delà de la longueur de ladite région dudit ancrage, impliquée dans la structure-tige.

5. Jeu d'amorces selon l'une quelconque des revendications 1-4, dans lequel ladite paire d'amorces comprend un ou plusieurs analogues d'oligonucléotides.

6. Jeu d'amorces selon l'une quelconque des revendications 1-5, dans lequel ladite amorce proprement dite comprend une ou plusieurs bases modifiées.

7. Jeu d'amorces selon la revendication 6, dans lequel ladite ou lesdites base(s) modifiée(s) est/sont une ou des base(s) universelle(s).

8. Jeu d'amorces selon la revendication 7, dans lequel ladite ou lesdites base(s) universelle(s) est/sont choisie(s) dans le groupe constitué d'inosine, de 5-nitroindole, de 3-nitropyrrole et de 4-nitrobenzimidazole.

9. Jeu d'amorces selon la revendication 6, dans lequel ladite ou lesdites base(s) modifiée(s) est/sont une ou des base(s) dégénérée(s).

10. Jeu d'amorces selon la revendication 9, dans lequel ladite ou lesdites base(s) dégénérée(s) est/sont choisie(s) dans le groupe constitué de 6H, de 8H-3, de 4-dihydropyrimido[4,5-c][1,2]oxazin-7-one (« P », un mimétique de pyrimidine) et de 2-amino-6-méthoxyaminopurine (« K », un mimétique de purine).

11. Jeu d'amorces selon l'une quelconque des revendications 1-10, dans lequel ledit ancrage comprend une ou plusieurs liaisons de squelette modifiées.

12. Jeu d'amorces selon l'une quelconque des revendications 1-11, dans lequel les séquences dudit ancrage et de ladite amorce ont chacune une longueur comprise entre 6 et 24 nucléotides.

13. Utilisation d'un jeu d'amorces selon l'une quelconque des revendications 1-12 pour l'amplification ou le séquençage d'une séquence polynucléotidique cible.

14. Procédé pour l'amplification ou le séquençage d'une séquence d'acide nucléique cible, comprenant les étapes:
- de combinaison d'un ancrage avant (FA), d'une amorce directe (FP), d'un ancrage inverse (RA), d'une amorce inverse (RP), d'une amorce universelle directe (FUP) et d'une amorce universelle inverse (RUP), où ledit FA et ladite FP s'auto-assemblent pour former une première paire d'oligonucléotide-amorce (FA/FP) et ledit RA et ladite RP s'auto-assemblent pour former une deuxième paire d'oligonucléotide-amorce (RA/RP) par une association de leurs régions complémentaires, où ladite FUP est complémentaire à la région impliquée dans la structure-tige de FA/FP, et ladite RUP est complémentaire à la région impliquée dans la structure-tige de RA/RP, et où lesdites paires d'oligonucléotide-amorces sont choisies sur la base de la complémentarité à ladite séquence d'acide nucléique cible;
- de combinaison desdites première et deuxième paires d'oligonucléotide-amorces et desdites FUP et RUP avec ledit acide nucléique cible; et
- d'amplification de ladite séquence d'acide nucléique cible par une amplification induite par une enzyme.

15. Procédé selon la revendication 14, dans lequel ladite séquence d'acide nucléique cible est un ADN.

16. Procédé selon la revendication 14, dans lequel ladite séquence d'acide nucléique cible est un ARN.

17. Procédé selon la revendication 16, dans lequel ladite amplification utilise une transcriptase inverse.

18. Procédé selon l'une quelconque des revendications 14-17, dans lequel ladite amplification induite par une enzyme est choisie dans le groupe constitué d'une amplification PCR, SDA, 3SR, IS-3SR, NASBA et du cercle roulant.

19. Procédé selon la revendication 18, dans lequel ladite amplification induite par une enzyme est une amplification PCR.

20. Procédé selon la revendication 18, dans lequel ladite amplification induite par une enzyme est choisie dans le groupe constitué d'une amplification 3SR, IS-3SR et NASBA.

21. Procédé selon la revendication 18, dans lequel ladite amplification induite par une enzyme est choisie dans le groupe constitué d'une amplification SDA et du cercle roulant.


### SEQUENCE LISTING

<110> OASIS BIOSCIENCES, INC.
   Brown, Bob D.
<120> AMPLIFICATION AND SEQUENCING PRIMER
   PAIRS AND USE THEREOF
<130> OASBIO.002VPC
<150> US 60/128,378
   <151> 1999-04-08
<160> 12
<170> FastSEQ for Windows Version 4.0
<210> 1
   <211> 19
   <212> DNA
   <213> Artificial Sequence
<220>
   <223> Synthetic oligonucleotide primers
<400> 1
   gccacctgtg gtccacctg 19
<210> 2
   <211> 14
   <212> DNA
   <213> Artificial Sequence
<220>
   <223> Synthetic oligonucleotide primers
<400> 2
   gccacctgtg gtcc 14
<210> 3
   <211> 31
   <212> DNA
   <213> Artificial Sequence
<220>
   <223> Synthetic oligonucleotide primers
<400> 3
   gccacctgtg gtcccacctg ctgaggtaga a 31
<210> 4
   <211> 36
   <212> DNA
   <213> Artificial Sequence
<220>
   <223> Synthetic oligonucleotide primers
<400> 4
   gccacctgtg gtccacctgc tgaggtagaa atctgg 36
<210> 5
   <211> 16
   <212> DNA
   <213> Artificial Sequence
<220>
   <223> Synthetic oligonucleotide primers
<400> 5
   ctgtctctca gcaggt 16
<210> 6
   <211> 22
   <212> DNA
   <213> Artificial Sequence
<220>
   <223> Synthetic oligonucleotide primers
<400> 6
   cgacgactgt ctctcagcag gt 22
<210> 7
   <211> 19
   <212> DNA
   <213> Artificial Sequence
<220>
   <223> Synthetic oligonucleotide primers
<400> 7
   cacttgtggc ccagatagg 19
<210> 8
   <211> 14
   <212> DNA
   <213> Artificial Sequence
<220>
   <223> Synthetic oligonucleotide primers
<400> 8
   cacttgtggc ccag 14
<210> 9
   <211> 30
   <212> DNA
   <213> Artificial Sequence
<220>
   <223> Synthetic oligonucleotide primers
<400> 9
   cacttgtggc ccagatagga ggctgcactc 30
<210> 10
   <211> 36
   <212> DNA
   <213> Artificial Sequence
<220>
   <223> Synthetic oligonucleotide primers
<400> 10
   cacttgtggc ccagatagga ggctgcactc cacgtc 36
<210> 11
   <211> 16
   <212> DNA
   <213> Artificial Sequence
<220>
   <223> Synthetic oligonucleotide primers
<400> 11
   catggtagcc tcctat 16
<210> 12
   <211> 21
   <212> DNA
   <213> Artificial Sequence
<220>
   <223> Synthetic oligonucleotide primers
<400> 12
   ttctaccatg gtagcctcct a 21